Europäisches Patentamt

(19) European Patent Office

Office européen des brevets

(11) Veröffentlichungsnummer: **0 071 140**
**B1**

(12) **EUROPÄISCHE PATENTSCHRIFT**

(45) Veröffentlichungstag der Patentschrift:
09.04.86

(21) Anmeldenummer: 82106477.1

(22) Anmeldetag: 19.07.82

(51) Int. Cl.⁴: **B 01 J 27/18,** C 07 C 51/25, C 07 C 57/145, B 01 J 27/198

(54) Vanadium/Phosphor-Mischoxid-Katalysator, Verfahren zu dessen Herstellung sowie dessen Verwendung.

(30) Priorität: 31.07.81 DE 3130343

(43) Veröffentlichungstag der Anmeldung:
09.02.83 Patentblatt 83/6

(45) Bekanntmachung des Hinweises auf die Patenterteilung:
09.04.86 Patentblatt 86/15

(84) Benannte Vertragsstaaten:
BE DE FR GB IT NL

(56) Entgegenhaltungen:
EP - A - 0 031 696
US - A - 4 016 105
US - A - 4 052 417
US - A - 4 171 316

(73) Patentinhaber: BAYER AG, Konzernverwaltung RP Patentabteilung, D-5090 Leverkusen 1 Bayerwerk (DE)

(72) Erfinder: Alpers, Heinz-Jürgen, Kastanienstrasse 21, D-4150 Krefeld 11 (DE)
Erfinder: Heller, Karl-Heinz, Dr., Scheiblerstrasse 103, D-4150 Krefeld (DE)
Erfinder: Lenz, Günter, Dr., Buschstrasse 165, D-4150 Krefeld (DE)

## Beschreibung

Die Erfindung betrifft einen Vanadium/Phosphor-Mischoxid-Katalysator, ein Verfahren zu dessen Herstellung sowie dessen Verwendung bei der Herstellung von Maleinsäureanhydrid.

Vanadium/Phosphor-Mischoxid-Katalysatoren sowie deren Verwendung bei der Herstellung von Maleinsäureanhydrid aus $C_4$-Kohlenwasserstoffen durch Gasphasen-Oxidation sind seit langem bekannt.

So wird bereits in der US-A 2 773 838 ein Vanadium/ Phosphor-Mischoxid-Katalysator beschrieben, mit dessen Hilfe ein Buten/Butan-Gemisch zu Maleinsäureanhydrid oxidiert wird (vgl. Beispiel II). Der Katalysator wird dabei durch Umsetzung von wäßriger Phosphorsäure mit Ammoniumvanadat ($NH_4VO_3$) hergestellt (vgl. Beispiel I). Bei der Oxidation des Buten/Butan-Gemisches werden Ausbeuten an Maleinsäureanhydrid erreicht, die maximal bei 55,8 Gew.-%, bezogen auf den eingesetzten Butenanteil, liegen (vgl. Tabelle II).

Um die Ausbeuten an Maleinsäureanhydrid mit Hilfe des Vanadium/Phosphor-Mischoxid-Katalysators noch zu steigern, wurde im Laufe der Zeit der Katalysator bzw. seine Herstellung auf verschiedenste Weise modifiziert. Es wurde z.B. versucht, die Aktivität und Selektivität des Vanadium/Phosphor-Mischoxid-Katalysators zu erhöhen durch Dotierung mit anderen Elementen, wie W, Sb, Nb und Mo (DE-A 27 50 327), W, Ni, Cd, Zn, Bi, Li, Cu, U, Zr, Hf, Cr, Fe, Mn, Mo und Co (DE-A 28 22 322), durch Veränderung der Wertigkeit des Vanadiums (US-PS 3 156 705; DE-OS 2 328 755), durch Umsetzung von vanadiumhaltigem Material mit o-Phosphorsäure in einer wasserfreien organischen Flüssigkeit in flüssiger Phase (DE-B 2 700 635 und US-A 4 244 879), durch Aktivierung eines Vanadium/Phosphor-Mischoxid-Katalysatorvorläufers mit Hilfe bestimmter Temperungsbedingungen (DE-A 2 256 909) oder durch Behandlung mit einer Säure, die stärker als Phosphorsäure ist, und anschließende Extraktion zur Entfernung löslicher Anteile (DE-A 2 822 322).

Bei allen diesen z.T. technisch aufwendigen Modifizierungsverfahren wurden Vanadium/Phosphor-Mischoxid-Katalysatoren erhalten, die nur unbefriedigende Ausbeuten an Maleinsäureanhydrid bei der Oxidation von $C_4$-Kohlenwasserstoffen liefern, was zu Lasten der Wirtschaftlichkeit der Maleinsäureanhydrid-Herstellungsverfahren geht.

Die Erfindung betrifft einen neuen Vanadium/Phosphor-Mischoxid-Katalysator, erhältlich durch Umsetzung einer Vanadium-V-Verbindung mit Phosphorsäure und/oder einer Phosphorsäure bildenden Verbindung in der Wärme, gegebenenfalls in Gegenwart von Wasser, gekennzeichnet durch eine anschließende 5 - 500-stündige Behandlung des nach der Umsetzung erhaltenen Produkts bei erhöhter Temperatur mit reduzierend wirkenden, organischen Lösungs- und/oder Verdünnungsmitteln, gegebenenfalls in Gegenwart eines Promotors.

Die Erfindung betrifft ferner ein Verfahren zur Herstellung eines Vanadium/Phosphor-Mischoxid-Katalysators bei dem man eine Vanadium-V-Verbindung mit Phosphorsäure und/oder einer Phosphorsäure bildenden Verbindung in der Wärme, gegebenenfalls in Gegenwart von Wasser, umsetzt, das dadurch gekennzeichnet ist, daß man anschließend das nach der Umsetzung erhaltene Produkt bei erhöhter Temperatur mit reduzierend wirkenden, organischen Lösungs- und/oder Verdünnungsmitteln, 5 - 500 Stunden lang, gegebenenfalls in Gegenwart eines Promotors, behandelt.

Weiterhin betrifft die Erfindung die Verwendung des Vanadium/Phosphor-Mischoxid-Katalysators zur Herstellung von Maleinsäureanhydrid aus $C_4$-Kohlenwasserstoffen in der Dampfphase.

Zur Herstellung des erfindungsgemäßen Vanadium/Phosphor-Mischoxid-Katalysators wird zunächst eine Vanadium-V-Verbindung mit Phosphorsäure oder einer Phosphorsäure bildenden Verbindung bei erhöhter Temperatur, gegebenenfalls in Gegenwart von Wasser, umgesetzt, wobei darauf zu achten ist, daß der Hauptteil der eingesetzten vanadium-verbindung in der 5-wertigen Stufe verbleibt. Aus diesem Grunde sollen bei der Umsetzung keine wesentlichen Mengen reduzierender Verbindungen zugegen sein. Auch dürfen die eingesetzten Vanadium- und Phosphorverbindungen keine reduzierenden Anionen enthalten. Das nach der Umsetzung erhaltene, aus Phosphaten des 5-wertigen Vanadiums bestehende Produkt, der sogenannte Katalysator-Vorläufer, besitzt ein Vanadium/Phosphor-Atomverhältnis von etwa 1:0,9 bis 1,5, vorzugsweise von 1:1,0 bis 1,3.

Im allgemeinen stellt man den Katalysator-Vorläufer so her, daß man die Vanadium-V-Verbindung und die Phosphorsäure oder die Phosphorsäure bildende Verbindung, gegebenenfalls in Gegenwart von Wasser, bei guter Durchmischung auf Temperaturen von etwa 80 bis 180° C, vorzugsweise 100 bis 150° C, ca. 1 bis 24 Stunden, bevorzugt 4 bis 8 Stunden, erhitzt.

Nach einer Herstellungs weise kann das Vermischen der Reaktionspartner in einem Kneter oder einer anderen üblichen Mischapparatur erfolgen, wobei man so viel Wasser zusetzen kann, daß eine fließfähige Paste entsteht. Üblicherweise setzt man dabei ca. 0 bis 30 Gew.-%, bevorzugt 5 bis 15 Gew.-%, Wasser, bezogen auf die Gesamtmischung, ein. Das dabei erhaltene Rohprodukt wird durch Eindampfen des pastenförmigen Reaktionsgemisches bis zur Trockene bei Temperaturen von etwa 100 bis 200° C isoliert.

Gemäß einer anderen Herstellungsweise werden die Vanadium-V-Verbindungen und die Phosphorsäure und/oder die Phosphorsäure bildenden Verbindungen unter Zusatz von etwa 30 bis 90 %, bevorzugt 40 bis 60 %, Wasser, bezogen auf die Gesamtmischung, in einer wäßrigen Suspension umgesetzt, wobei sich das Reaktionsprodukt in kristalliner Form abscheidet. Das Reaktionsprodukt wird anschließend abfiltriert, zweckmäßigerweise mit Wasser und anschließend mit Aceton gewaschen und dann bei ca. 100 bis 180° C getrocknet.

Als Vanadium-V-Verbindungen können z.B. Vanadiumpentoxid, Ammoniumvanadat und/oder Natriumvanadat eingesetzt werden. Bevorzugt wird VanadiumPentoxid eingesetzt.

Als Phosphorsäure bildende Verbindungen können für das erfindungsgemäße Verfaren Phosphorpentoxid, m-

2

Phosphorsäure und/oder Polyphosphorsäuren eingesetzt werden. Bei Einsatz von Phosphorsäure bildenden Verbindungen kann es zweckmäßig sein, die Umsetzung in Gegenwart von Wasser durchzuführen. Dabei ist es vorteilhaft, so viel Wasser zuzugeben, daß sich die Phosphorsäure bildenden Verbindungen nahezu vollständig in Phosphorsäure umwandeln.

Die zur Herstellung des Katalysator-Vorläufers eingesetzte Menge an Phosphorsäuren und/oder Phosphorsäure bildender Verbindung, bezogen auf die eingesetzte Menge an Vanadium-Verbindung, hängt von der Herstellungsweise ab.

Wird der feste Vorläufer aus dem Reaktionsgemisch durch Eindampfen zur Trockene gewonnen, so ist das Atomverhältnis Vanadium zu Phosphor gleich dem des fertigen Vorläufers, nämlich 1:0,9 bis 1:1,5, vorzugsweise 1:1,0 bis 1:1,3.

Wird dagegen der feste Katalysator-Vorläufer durch Filtrieren aus der Reaktionsmischung abgetrennt, so kann ein Überschuß an Phosphorsäure verwendet werden. Dabei kann das Atomverhältnis Vanadin zu Phosphor des Einsatzgemisches etwa 1:0,9 bis 1:30, vorzugsweise 1:3 bis 15, betragen.

Der erhaltene Katalysator-Vorläufer wird anschließend, gegebenenfalls in gemahlener Form, bei erhöhter Temperatur (etwa 80 bis 200° C, bevorzugt 90 bis 120° C) in suspendierter Form mit reduzierend wirkenden organischen Lösungs- und/oder Verdünnungsmitteln, gegebenenfalls in Gegenwart von zusätzlicher Phosphorsäure und gegebenenfalls in Gegenwart von Katalysatorpromotoren, 5 bis 500 Stunden lang behandelt, wobei man die während der Behandlung entstehenden, geringen Mengen Wasser gegebenenfalls aus dem Reaktionsgemisch, beispielsweise durch Destillation, entfernt. Bevorzugt wird hierbei nur die Hauptmenge Wasser entfernt. Jedoch ist es ebenso möglich, die Behandlung des Katalysator-Vorläufers mit reduzierenden Lösungs- oder Verdünnungsmitteln in Gegenwart von vorher zugefügtem Wasser durchzuführen.

Als organische Lösungs- und/oder Verdünnungsmittel kommen polare, Kohlenstoff-, Sauerstoff- und Wasserstoff-haltige Verbindungen, die 1 bis 10, bevorzugt 3 bis 6, Kohlenstoffatome im Molekül enthalten, in Frage, wie aliphatische, cycloaliphatische oder araliphatische, gesättigte oder ungesättigte Alkohole, Aldehyde, Ketone, Säuren und Ester, insbesondere Alkohole, Aldehyde und Säuren.

Zum Beispiel werden als Alkohole: Propanol-(1), Propanol-(2), 2-Methyl-propanol-(1), Butanol-(1), Pentanole, Hexanole, Cyclohexanol und Benzylalkohol, bevorzugt Butanol-(1), 2-Methyl-propanol-(1), als Aldehyde: Propanal, Butanal, 2-Methylpropanal, Pentanal, Hexanal und Benzaldehyd, bevorzugt Butanal, 2-Methylpro-panal und Pentanal, als Ketone: Aceton, Butanon, Pentanon und Cyclohexanon, bevorzugt Pentanon, als Säuren: Ameisensäure, Essigsäure, Propionsäure, Oxalsäure, Citronensäure und Ascorbinsäure, bevorzugt Essigsäure und Oxalsäure, und als Ester: Ethylacetat, Propylacetat, Butylacetat, Ethylpropionat und Butylpropionat, bevorzugt Butylacetat, eingesetzt.

Die organischen Lösungs- und/oder Verdünnungsmittel können sowohl einzeln als auch im Gemisch untereinander eingesetzt werden. Wenn das organische Lösungsund/oder Verdünnungsmittel selbst keine reduzierende Eigenschaft besitzt, dann ist es nur im Gemisch mit einem anderen reduzierend wirkenden organischen Lösungs- und/oder Verdünnungsmittel einzusetzen.

Im allgemeinen setzt man die organischen Lösungsund/oder Verdünnungsmittel in Mengen von etwa 0,5 bis 20, bevorzugt 2 bis 10 Teilen, bezogen auf 1 Teil des Katalysator-Vorläufers, ein.

Die Menge der gegebenenfalls bei der Behandlung des Katalysator-Vorläufers mit den organischen Lösungsund/oder Verdünnungsmitteln eingeseuzten Phosphorsäure, die üblicherweise in Form einer etwa 85 bis 100 gew.-%igen, bevorzugt 100 gew.-%igen, Phosphorsäure eingesetzt wird, beträgt ca. 0,01 bis 0,5, bevorzugt 0,1 bis 0,2 Teile, bezogen auf 1 Teil des Vorläufers.

Als Katalysatorpromotoren kommen beispielsweise die Elemente der I., II., VII. und/oder VIII. Nebengruppe des Periodensystems der Elemente (Mendelejew) in Frage. Beispielsweise werden genannt: Kupfer, Silber, Zink, Cadmium, Mangan, Eisen, Kobalt, Nickel, Palladium und/oder Platin, bevorzugt Eisen und/oder Mangan.

Die Katalysatorpromotoren können in elementarer Form oder in Form ihrer Verbindungen, wie den Carbonaten, Hydrogencarbonaten, Phosphaten, Acetaten und/oder den Oxalaten, bevorzugt den Phosphaten und/oder den Oxalaten, eingesetzt werden.

Die Menge der einzusetzenden Promotoren wird vorteilhafterweise so bemessen, daß das Atomverhältnis von Promotor zu Vanadium etwa 0,01 bis 0,1, bevorzugt 0,02 bis 0,07, beträgt.

Es ist selbstverständlich auch möglich, die Katalysatorpromotoren statt bei der Behandlung des Katalysator-Vorläufers schon bei dessen Herstellung oder nach her Behandlung des vorläufers mit organischen Lösungs- und/oder Verdünnungsmitteln dem fertigen Vanadium/Phosphor-Mischoxid-Katalysator zuzugeben.

Eine Möglichkeit der Behandlung des Katalysator-Vorläufers mit einem organischen Lösungs- und/oder Verdünnungsmittel besteht darin, daß man den gegebenenfalls gemahlenen Vorläufer mit Phosphorsäure und dem organischen Lösungs- und/oder Verdünnungsmittel, gegebenenfalls unter Zusatz eines Promotors, bei Temperaturen von ca. 80 bis 200° C, bevorzugt 90 bis 120° C, bei guter Durchmischung erhitzt, wobei man die während der Behandlung gebildeten geringen Mengen an Wasser aus dem Reaktionsgemisch in üblicher Weise, gegebenenfalls unter Verwendung von mit Wasser nicht mischbaren, aliphatischen und/oder aromatischen Kohlenwasserstoffen, wie Pentan, Cyclohexan, Toluol und/oder Xylol, entfernt (Azeotropdestillation). Zur besseren Wasserabtrennung kann auch eine Destillierkolonne mit 1 bis 10 theoretischen Böden verwendet werden.

Nach 5- bis 500-, bevorzugt 10- bis 50-stündiger Behandlung des Katalysator-Vorläufers, der nunmehr in einer niedrigeren Wertigkeitsstufe (mittlere Wertigkeit des Vanadiums liegt bei etwa 3,9 bis 4,2) vorliegt, kann die Suspension entweder bei Temperaturen von etwa 80 bis 150° C zur Trockne eingedampft werden oder der

3

Feststoff aus der Suspension durch Filtrieren abgetrennt werden. Dabei kann das zurückerhaltene organische Lösungs- und/oder Verdünnungsmittel für weitere Ansätze wiederverwendet werden.

Der abfiltrierte Feststoff wird vorteilhafterweise bei ca. 100 bis 150° C in Gegenwart von Luft oder Stickstoff oder im Vakuum getrocknet.

Der erhaltene Vanadium/Phosphor-Mischoxid-Katalysator kann anschließend durch Tempern bei Temperaturen bis zu etwa 600° C, bevorzugt bei 350 bis 450° C, in üblicher Weise aktiviert und dann in eine für den technischen Einsatz geeignete Form, z.B. durch Verpressen zu geformten Katalysatorteilchen oder Einstreichen in Lochbleche, gegebenenfalls unter Zusatz von inerten Verschnittmitteln, wie Kieselsäure, Aluminiumoxid und/oder Titandioxid, gebracht werden. Selbstverständlich ist es auch möglich, die Katalysatormasse auf die üblichen inerten Träger, wie Aluminiumoxid, Siliciumcarbid oder Steatit, mit bekannten Methoden aufzubringen.

Der Vanadin-Phosphor-Mischoxid-Katalysator kann in bekannter Weise für die Oxidation von geradkettigen $C_4$-Kohlenwasserstoffen, wie Buten-1, cis- und trans-Buten-2 und/oder n-Butan oder einer Mischung hieraus, bevorzugt Buten und Buten/Butan-Gemischen, mit Luft zu Maleinsäureanhydrid verwendet werden (vgl. hierzu z.B. Ullmann, Enzyklopädie der Technischen Chemie, Band 9, S. 147 f., Band 16, S. 408 ff).

Wie die in den Beispielen der vorliegenden Anmeldung aufgeführten Vergleichsversuche, die unter technischen Bedingungen mit bekannten Vanadium/Phosphor-Mischoxid-Katalysatoren, die u.a. gemäß der DE-B-2 700 635 und der DE-A- 2 822 322 hergestellt und in eine für den technischen Einsatz geeigneten Form gebracht wurden, zeigen, wird durch den erfindungsgemäß hergestellten Vanadium/Phosphor-Mischoxid-Katalysator eine verbesserte Selektivität, verbunden mit einer beträchtlichen Ausbeutesteigerung an Maleinsäureanhydrid von über 10 Gew.-%, bezogen auf den anfänglich eingesetzten $C_4$-Kohlenwasserstoff, erzielt.

Die nachfolgenden Beispiele sollen die erfindungsgemäße Katalysatorherstellung und die Verwendung des erfindungsgemäßen Katalysators bei der Maleinsäureanhydridherstellung verdeutlichen.

**Beispiel 1**

a) Herstellung des Katalysatorvorläufers (Präkursors); Ansatz:

1 kg Vanadinpentoxid $V_2O_5$

9 kg 85 %ige Phosphorsäure ($H_3PO_4$)

8 kg Wasser

Wasser und Phosphorsäure wurden zum Sieden erhitzt und das Vanadinpentoxid wurde danach unter Rühren eingetragen. Nach 8 Stunden Rühren und Kochen am Rückfluß wurde auf 20° C abgekühlt, abfiltriert, mit Wasser und danach mit Aceton gut ausgewaschen, um anhaftende überschüssige Phosphorsäure zu entfernen. Danach wurde das Produkt bei 150° C getrocknet.

b) Nachbehandlung des Katalysatorvorläufers mit iso-Butanol/n-Butanal;

Ansatz:

1 kg Katalysatorvorläufer (trocken)

121 g Phosphorsäure (100%ig)

3,7 kg iso-Butanol

480 g Butanal

Die Komponenten wurden zusammen unter Rühren am Rückfluß gekocht. Die entstehenden Dämpfe wurden über eine Destillierkolonne und einen Kondensator einem Wasserabscheider zugefürt. Die Länge der verwendeten Destillierkolonne soll 1 bis 2 theoretischen Stufen entsprechen. Während der Kochzeit von insgesamt 50 Stunden wurden im Wasserabscheider 50 bis 100 ml Wasser erhalten und abgetrennt. Nach dem Ende der Kochzeit wurde abgekühlt auf ca. 20° C und abfiltriert. Das erhaltene Rohprodukt wurde unter Stickstoff bei 100° C getrocknet.

c) Weiterverarbeitung des behandelten Katalysator-Präkursors zu Katalysatorkugeln

Das unter b) erhaltene getrocknete Produkt wurde mit einer Aufheizrate von 150° C/h von ca. 20° C auf 410° C aufgeheizt und 5 h auf dieser Temperatur gehalten. Das so getemperte Produkt wurde gemahlen und unter Zusatz von ca. 6 % Aluminiumstearat zu Kugeln von 6,6 mm Ø verpreßt.

Der Katalysator besitzt ein Atomverhältnis von Vanadium zu Phosphor von 1:1,2.

**Beispiel 2**

Die Durchführung entsprach Beispiel 1, aber bei der Nachbehandlung wurden dem Ansatz 55,5 g Eisenoxalat ($FeC_2O_4 \times 2H_2O$) zugegeben. Das Vanadium/Eisen-Atomverhältnis entspricht 1:0,05.

**Beispiel 3**

Das Rohprodukt wurde hergestellt wie unter Beispiel 1 beschrieben, aber bei der Nachbehandlung wurden dem Ansatz 222 g Eisenoxalat zugegeben (Atomverhältnis Vanadium zu Eisen wie 1:0,2).

**Beispiel 4**

a) Herstellung des Katalysatorvorläufers; Ansatz:
736 g $V_2O_5$
1118 g $H_3PO_4$ (85 %ig)
76 g Eisenoxalat
Die Ausgangsstoffe wurden in einem Kneter ohne Verwendung weiterer Lösungsmittel 3 Stunden gemischt, auf 120° C erwärmt und 2 h bei dieser Temperatur gehalten.
b) Nachbehandlung des Katalysatorvorläufers mit iso-Butanol/n-Butanal;
Ansatz:
1450 g Präkursor aus Stufe a)
3,7 kg iso-Butanol
480 g n-Butanal
Die weitere Behandlung erfolgte wie unter Beispiel 1 beschrieben (Atomverhältnis Vanadium zu Eisen wie 1:0,05 und Vanadium zu Phosphor wie 1:1,2).

**Beispiel 5**

Die Durchführung entsprach Beispiel 1, aber bei der Nachbehandlung wurden dem Ansatz 35,5 g $MnCO_3$ zugesetzt (Atomverhältnis Vanadium zu Mangan wie 1:0,05).

**Beispiel 6**

Die Durchführung entsprach Beispiel 1, aber bei der Nachbehandlung wurden dem Ansatz 55,5 g Eisenoxalat ($FeC_2O_4$ x $2H_2O$) zugegeben und die Nachbehandlung wurde mit einem Gemisch aus 3,7 kg n-Butanol und 480 g Butanal durchgeführt.

**Beispiel 7**

Die Durchführung entsprach Beispiel 6, mit der Abänderung, daß die Nachbehandlung mit einem Gemisch aus 3,7 kg iso-Butanol und 700 g Benzylalkohol durchgeführt wurde.

**Biespiel 8**

Die Durchführung entsprach Beispiel 6, mit der Abänderung, daß die Nachbehandlung mit 4,2 kg iso-Butanol durchgeführt wurde und auf 300 Stunden verlängert wurde.

**Beispiel 9**

Die Durchführung entsprach Beispiel 6, mit der Abänderung, daß die Nachbehandlung mit einem Gemisch aus 3,7 kg Eisessig und 600 g Butanal durchgeführt wurde und daß bei der Nachbehandlung kein Wasserabscheider verwendet wurde.

# 0 071 140

**Beispiel 10**

(Vergleichsbeispiel, ohne erfindungsgemäße Nachbehandlung des Katalysator-Vorläufers)
Ansatz:
1100 g Katalysatorvorläufer mit 10 % Restwasser, hergestellt nach Beispiel 1 a), aber ohne vollständige Trocknung.
117 g 85 %ige $H_3PO_4$
2,5 l Wasser
48 g $FePO_4$
Die Komponenten wurden gemischt und 4 Stunden unter Rühren am Rückfluß gekocht. Danach wurde auf 20° C abgekühlt, abgesaugt und der Rückstand bei 150° C getrocknet. Die Weiterverarbeitung erfolgte wie unter Beispiel 1c beschrieben (Atomverhältnis V:P:Fe wie 1:1,2:0,5).

**Beispiel 11**

(nach DE-B- 2 700 635/Vergleichsbeispiel)
Ansatz:
693 g $V_2O_5$ (Komponente 1)
2700 ml iso-Butanol (Komponente 2)
900 ml Benzylalkohol (Komponente 3)
900 g Phosphorsäure (Komponente 4)
900 ml iso-Butanol (Komponente 5)
Apparatur:
6 l Kolben mit Rührwerk, Tropftrichter für Zugaben und Kühler mit Wasserabscheider.
Durchführung:
Die Komponenten 1, 2, 3 wurden in der Apparatur vorgelegt und 5 h unter Rückfluß gerührt. Danach wurde aut 60° C abgekühlt und die Lösung von Komponente 4 in Komponente 5 langsam zugesetzt. Danach wurde noch 20 h unter Rückfluß gerührt. Nach dem Abkühlen auf Raumtemperatur wurde der Feststoff abfiltriert und getrocknet. Die Weiterverarbeitung erfolgte wie unter Beispiel 1c beschrieben (Atomverhältnis V:P wie 1:1,2).

**Beispiel 12**

nach DE-B 2 700 635/Vergleichsbeispiel)
Ansatz:
772,5 g $V_2O_5$ (Komponente 1)
64,05 g $FePO_4$ (Komponente 2)
2000 ml iso-Butanol (Komponente 3)
1000 ml Benzylalkohol (Komponente 4)
958 g Phosphorsäure (Komponente 5)
2000 ml iso-Butanol (Komponente 6)
Apparatur wie unter Beispiel 7 beschrieben.
Durchführung:
Die Komponenten 1, 2, 3, 4 wurden unter Rühren und Rückfluß 22 h gekocht, Danach wurde auf Raumtemperatur abgekühlt und die Lösung von Komponente 5 in Komponente 6 langsam zugegeben. Danach wurde nochmals 20 h unter Rückfluß gekocht. Nach Abkühlen auf 20° C wurde abfiltriert, getrocknet und wie in Beispiel 1c weiterverarbeitet Atomverhältnis V:P:Fe wie 1:1, 2:0,05.)

**Beispiel 13**

(nach DE-B 2 700 635/Vergleichsbeispiel)
Ansatz:
772,5 g $V_2O_5$ (Komponente 1)
256,2 g $FePO_4$ (Komponente 2)
2000 ml iso-Butanol (Komponente 3)
1000 ml Benzylalkohol (Komponente 4)
833 g Phosphorsäure (Komponente 5)
2000 ml iso-Butanol (Komponente 6)
Durchführung wie unter Beispiel 12 beschrieben (Atomverhältnis V:P:Fe wie 1:1,2:0,2).

6

**Beispiel 14**

(nach DE-A- 2 822 322/Vergleichsbeispiel)
a) 606 g Vanadinpentoxid wurden mit 7900 ml konzentrierter Salzsäure 2 h unter Rückfluß gekocht.
Zu der erhaltenen dunkelblauen Lösung wurden 891 g 88 %ige Phosphorsäure gegeben und die Lösung nach 2 h Kochen am Rückfluß auf 2000 ml Restvolumen eingeengt.
Der Rückstand wurde mit 2000 ml konzentrierter Salzsäure 1 h am Rückfluß gekocht, dann eingedampft und der erhaltene Feststoff bei 110° C getrocknet.
b) Das getrocknete Produkt wurde mit Wasser (20 ml/g) 1 Stunde gekocht und heiß filtriert. Der Filterrückstand wurde mit wenig Wasser gewaschen und bei 150° C getrocknet.
Die Weiterverarbeitung erfolgte wie unter Beispiel 1c beschrieben (Atomverhältnis V:P wie 1:1,2).

**Beispiel 15**

(nach DE-A 2 822 322/Vergleichsbeispiel)
Feststoff, der analog Beispiel 10 hergestellt worden war, wurde mit iso-Butanol (20 ml/g) 1 Stunde gekocht, heiß filtriert, mit wenig iso-Butanol gewaschen und bei 150° C getrocknet. Die Weiterverarbeitung erfolgte wie unter Beispiel 1c beschrieben (Atomverhältnis V:P wie 1:1,2).

**Verwendung der Vanadium/Phosphor-Mischoxid-Katalysatoren**

Die Versuche sollen Aussagen über das Verhalten bei der technischen Herstellung von Maleinsäureanhydrid liefern. Es wurden deshalb Einrohrreaktoren benutzt, die in ihren Abmessungen und ihrem Verhalten den technischen Reaktoren entsprechen.
Abmessungen der Reaktionsrohre:
25 mm Durchmesser
3500 mm Länge
2500 mm Kontaktschüttung
Der Kontakt befand sich in den Rohren; die Rohre waren von einer Salzschmelze umgeben, deren Temperatur variiert werden konnte. Verwendet wurde jeweils 1 l des zu prüfenden Kontaktes. Als Einsatzstoff wurde ein Gemisch aus $C_4$-Kohlenwasserstoffen folgender (typischer) Zusammensetzung verwendet:
19,7 % n-Butan
4,5 % i-Butan
42,3 % n-Buten-(1)
13,7 % cis-Buten-(2)
19,3 % trans-Buten-(2)
Aus dem eingesetzten Kohlenwasserstoffgemisch und Luft wurde ein Reaktionsgas mit 1,5 Vol.-% Kohlenwasserstoff hergestellt, 2,7 $m^3_n$/h dieses Gases wurden durch die Kontaktschüttung geleitet (Raumgeschwindigkeit = 2700 $h^{-1}$). Unter Raumgeschwindigkeit (space velocity) ist das pro Katalysatorvolumen durchgesetzte Gasvolumen unter Normalbedingungen zu verstehen. Durch Waschen des Austrittsgases mit Wasser wurde das entstandene Maleinsäureanhydrid (dann in der hydratisierten Form als Maleinsäure) aufgefangen und seine Menge durch potentiometrische Titration bestimmt. Durch Verändern der Temperatur der Salzschmelze wurde die optimale Salzbadtemperatur gesucht; d.h. die Temperatur, bei der die maximale Ausbeute an MSA erhalten wurde. Die in der folgenden Tabelle angegebenen Ausbeutewerte bedeuten die Maleinsäureanhydrid (MSA)-Ausbeute in Gew.-%, bezogen auf eingesetzte Butene.

**Zusammenstellung der erhaltenen MSA-Ausbeuten**

| Beispiel | Vanadium/ Phosphor- Mischoxid- Katalysator Atomverhältnisse V:P:Zusatz | | | Badtempera- tur für die Umsetzung zu MSA (°C) | MSA- Ausbeute (Gew.-%) |
|---|---|---|---|---|---|
| 1 | 1 1,2 | - | | 450 | 94 |
| 2 | 1 1,2 | 0,05 | Fe | 430 | 103 |
| 3 | 1 1,2 | 0,20 | Fe | 460 | 78 |
| 4 | 1 1,2 | 0,05 | Fe | 450 | 100 |
| 5 | 1 1,2 | 0,05 | Mn | 450 | 100 |
| 6 | 1 1,2 | 0,05 | Fe | 400 | 86 |
| 7 | 1 1,2 | 0,05 | Fe | 420 | 97 |
| 8 | 1 1,2 | 0,05 | Fe | 420 | 100 |
| 9 | 1 1,2 | 0,05 | Fe | 410 | 97 |

Vergleichsbeispiele

| | | | | | |
|---|---|---|---|---|---|
| 10 | 1 1,2 | 0,05 | Fe | 450 | 55 |
| 11* | 1 1,2 | | | 450 | 82 |
| 12* | 1 1,2 | 0,05 | Fe | 430 | 83 |
| 13* | 1 1,2 | 0,20 | Fe | 460 | 64 |
| 14** | 1 1,2 | | | 450 | 76 |
| 15** | 1 1,2 | | | 450 | 80 |

\* Nr. 11,12,13 nach DE-AS 2 700 635

\*\* Nr. 14,15 nach DE-AS 2 822 322

**Erklärung zur Zusammenstellung:**

Vergleichsbeispiel Nr. 10 (ohne Nachbehandlung)
Vergleichsbeispiel Nr. 11 (zu Beispiel 1)
Vergleichsbeispiel Nr. 12 (zu Beispielen 2, 4, 6-9)
Vergleichsbeispiel Nr. 13 (zu Beispiel 3)
Vergleichsbeispiel Nr. 14 (zu Beispiel 1)
Vergleichsbeispiel Nr. 15 (zu Beispiel 1)

**Beispiel 16**

Der nach Beispiel 4 hergestellte Kontakt wurde nach dem oben beschriebenen Testverfahren geprüft, wobei aber die Eintrittskonzentration des Kohlenwasserstoffs 1,95 Vol% betrug. Bei einer optimalen Badtemperatur von 410° C betrug die wie oben definierte Ausbeute an MSA 101 %.

**Patentansprüche**

1) Vanadium/Phosphor-Mischoxid-Katalysator, erhältlich durch Umsetzung einer Vanadium-V-Verbindung mit Phosphorsäure und/oder einer Phosphorsäure bildenden Verbindung in der Wärme, gegebenenfalls in Gegenwart von Wasser, gekennzeichnet durch eine anschließende 5 - 500-stündige Behandlung des nach der Umsetzung erhaltenen Produkts bei erhöhter Temperatur mit reduzierend wirkenden, organischen Lösungs- und/oder Verdünnungsmitteln, gegebenenfalls in Gegenwart eines Promotors.

2) Verfahren zur Herstellung eines Vanadium/Phosphor-Mischoxid-Katalysators, bei dem man eine Vanadium-V-Verbindung mit Phosphorsäure und/oder einer Phosphorsäure bildenden Verbindung in der Wärme, gegebenenfalls in Gegenwart von Wasser, umsetzt, dadurch gekennzeichnet, daß man anschließend das nach der Umsetzung erhaltene Produkt bei erhöhter Temperatur mit reduzierend wirkenden, organischen Lösungs- und/oder Verdünnungsmitteln, 5 - 500 Stunden lang, gegebenenfalls in Gegenwart eines Promotors, behandelt.

3) Verfahren nach Anspruch 2, dadurch gekennzeichnet, daß man die anschließende Behandlung des nach der Umsetzung erhaltenen Produkts bei 80 bis 200°C durchführt.

4) Verfahren nach Ansprüchen 2 und 3, dadurch gekennzeichnet, daß man als organische Lösungs- und/oder Verdünnungsmittel polare, Kohlenstoff-, Sauerstoffund Wasserstoff-haltige Verbindungen, die 1 bis 10 Kohlenstoffatome im Molekül enthalten, einsetzt.

5) Verfahren nach Ansprüchen 2 bis 4, dadurch gekennzeichnet, daß man als organische Lösungs- und/oder Verdünnungsmittel aliphatische, cyclcaliphatishe oder araliphatische, gesättigte oder ungesättigte Alkohole, Aldehyde, Ketone, Säuren und/oder Ester mit 1 bis 10 Kohlenstoffatomen einsetzt.

6) Verfahren nach Ansprüchen 2 bis 5, dadurch gekennzeichnet, daß man als Katalysator-promotoren die Elemente der I., II., VII. und/oder VIII. Nebengruppe des Periodensystems der Elemente (Mendelejew) einsetzt.

7) Verwendung des Vanadium/Phosphor-Mischoxid-Katalysators gemäß Anspruch 1 zur Herstellung von Maleinsäureanhydrid aus $C_4$-Kohlenwasser-stoffen in der Dampfphase.

**Claims**

Vanadium/phosphorus mixed oxide catalyst, obtainable by reaction of a vanadium V compound with phosphoric acid and/or a compound which forms phosphoric acid with heating, optionally in the presence of water, characterised by a subsequent 5 - 500-hour treatment of the product obtained after the reaction at an elevated temperature with organic solvents and/or diluents having reducing action, optionally in the presence of a promoter.

2. Process for the preparation of a vanadium/phosphorus mixed oxide catalyst, in which a vanadium V compound is reacted with phosphoric acid and/or a compound which forms phosphoric acid with heating, optionally in the presence of water, characterised in that the product obtained after the reaction is subsequently treated at an elevated temperature, for 5 - 500 hours, with organic solvents and/or diluents having reducing action, octionally in the presence of a promoter.

3. Process according to Claim 2, characterised in that the subsequent treatment of the product obtained after the reaceion is carried out at 80 to 200°C

4. Process according to Claims 2 and 3, characterised in that polar compounds containing carbon, oxygen and hydrogen, which contain 1 to 10 carbon atoms in the molecule, are used as the organic solvents and/or diluents.

5. Process according to Claims 2 to 4, characterised in that aliphatic, cycloaliphatic or araliphatic, saturated or unsaturated alcohols, aldehydes, ketones, acids and/or esters with 1 to 10 carbon atoms are used as the organic solvents and/or diluents.

6. Process according to Claims 2 to 5, characterised in that the elements of subgroups I, II, VII and/or VIII of the periodic system of the elements (Mendelejev) are used as the catalyst promoters.

7. Use of the vanadium/phosphorus mixed oxide catalyst according to Claim 1 for the preparation of maleic anhydride from $C_4$ hydrocarbons in the vapour phase.

**Revendications**

1. Catalyseur d'oxyde mixte vanadium/phosphore, obtenable par réaction d'un composé de vanadium-V avec de l'acide phosphorique et/ou un composé formateur d'acide phosphorique à chaud, éventuellement en présence d'eau, caractérisé par un traitement consecutif de 5 à 500 heures, du produit obtenu après la réaction, à une température élevée avec des solvants et/ou diluants organiques à action réductrice, éventuellement en présence d'un promoteur.

2. Procédé de fabrication d'un catalyseur d'oxyde mixte vanadium/phosphore, dans lequel on fait réagir un composé de vanadium-V avec de l'acide phosphorique et/ou un composé formateur d'acide phosphorique à chaud, éventuellement en présence d'eau, caractérisé en ce que par la suite le produit, obtenu après la réaction, est traite à température élevée avec des sovlants et/ou diluants organiques à action réductrice, durant 5 à 500 heures, éventuellement en présence d'un promoteur.

3. Procédé selon la revendication 2, caractérisé en ce qu'on exécute le traitement consécutif du produit obtenu après la réaction à 80 - 200°C.

4. Procédé selon les revendications 2 et 3, caractérisé en ce qu'on utilise comme solvants et/ou diluants organiques des composés polaires contenant du carbone, de l'oxygène et de l'hydrogène, qui contiennent 1 à 10 atomes de carbone dans la molécule.

5. Procédé selon les revendications 2 à 4, caractérisé en ce qu'on utilise comme solvants et/ou diluants organiques des alcools, aldéhydes, cétones, acides et/ou esters aliphatiques, cycloaliphatiques ou araliphatiques, saturés ou non saturés, ayant 1 à 10 atomes de carbone.

6. Procédé selon les revendications 2 à 5, caractérisé en ce qu'on utilise comme promoteurs de catalyseur les éléments du Ier, IIe, VIIe et/ou VIIIe sous-groupe du système périodique des éléments (Mendelejew).

7. Utilisation du catalyseur d'oxyde mixte vanadium/phosphore selon la revendication 1 pour la fabrication d'anhydride maléique à partir d'hydrocarbures en $C_4$ en phase vapeur.